# EUROPEAN PATENT SPECIFICATION

(11) **EP 1 688 117 B1**
(45) Date of publication and mention of the grant of the patent: **27.11.2013**
(21) Application number: 05102923.9
(22) Date of filing: 13.04.2005
(51) Int. Cl.: A61F 13/56, A61F 13/62

(54) **Absorbent article comprising a fastening system**
Absorbierender Artikel umfassend ein Befestigungssystem
Article absorbant comprenant un système de fixation

(30) Priority: 08.02.2005 EP 05002584
(43) Date of publication of application: 09.08.2006
(73) Proprietor: Attends Healthcare AB, 578 24 Aneby (SE)
(72) Inventor: Kvarnström, Irina, 573 39, TRANAS (SE); Häkansson, Mikael, 573 37, TRANAS (SE); Persson, Christer, 578 31, ANEBY (SE); Lindqvist, Arne, 151 35, SÖDERTÄLJE (SE)
(74) Representative: Edlund, Fabian

(56) References cited:
- WO-A-02/22064
- US-A- 5 624 429
- US-A1- 2003 100 880
- US-A1- 2004 236 301

## Description

### Field of the Invention

The present invention discloses an absorbent article, such as an incontinence guard, comprising a front portion, a rear portion and a crotch region there between, and further being provided with a pair of band portions propagating from the rear portion and adapted to join said front portion from respective side of the waist when said absorbent article is worn by a wearer.

### Technical Background

Many of the known absorbent articles have a tendency to sag, slip, slide or gap away from the body of the wearer during use. This sagging, slipping, sliding or gapping is caused by the relative motions of the wearer during use. It is also caused by the downward forces generated when the absorbent article is loaded with body exudates and by the deformation of the materials of the absorbent article itself when subjected to such wearer's motions. The sagging/gapping and sliding/slipping can lead to premature leakage and poor fit of the absorbent article about the wearer.

In addition to addressing problems with the fit and sustained fit of absorbent articles, much attention has been focused on comfort. EP 0 799 002 discloses an absorbent article having closure means comprising a pair of spaced apart mechanical fasteners (A) disposed along the back waist region of the containment assembly, said mechanical fasteners being disposed substantially parallel to the longitudinal axis of said containment assembly and complimentary mechanical fastener landing surfaces (B) disposed along the front waist portion of the containment assembly, whereby upon properly attaching the absorbent article to a wearer, the mechanical fasteners engage the mechanical fastener landing surfaces to assist in forming said plane of tension together with a pair of fastening tabs (C) extending at a downward angle. However, in order to provide a good enough fit for wearers of different sizes and shapes these kind of covering absorbent articles are suitably provided in a substantial amount of alternative embodiments in order to be used optimally.

WO 02/22064 discloses a belted absorbent article, comprising a pair of belt portions, which are intended to be attached around the waist of a wearer, before attaching the body facing surface of the front portion of the absorbent article to the belt portions.

US 2004/236301 discloses an absorbent article, in the form of a baby diaper, with a reconnectable fastening element.

US 2003/100880 discloses an absorbent article comprising band portions with two fastening elements arranged along the band portions at different locations.

US 5 624 429 discloses a baby diaper which is provided with band portions having fixing zones of different fastening strenghts.

WO 03/070136 presents a fastening system for securing a first waist region to a second waist region for providing a first and a second fit, wherein the first fit provides a loose fit enabling the article to be manoeuvred about the wearer's waist, the fastening system including instructional serviceable indicia providing information corresponding to the first and second fit. According to this solution, which represents a more conventional fastening system, a more flexibly adjustable product is accomplished, although the issue of sagging is not addressed. Hence, it is desirable to present a solution for a more conventional system that provides for adjustability, is resistant to sagging and yet comfortable. A further object is to provide an improved absorbent article fastening system and a method for applying an absorbent article in an efficient manner and yet fully utilizing the benefits of the improved absorbent article.

### Summary of the invention

The object of the present invention is to overcome the above issues and provide an improved absorbent article fastening system. According to the invention there is provided an absorbent article, such as an incontinence guard, comprising a front portion, a rear portion and a crotch region there between, and further being provided with a pair of band portions propagating from the rear portion and adapted to join said front portion from respective side of the waist when said absorbent article is worn by a wearer, said each band portion being reconnectably joinable to said front portion in a respective first attachment zone of said absorbent article by a first fastening means, said each band portion being joinable by a second fastening means in a respective second attachment zone, said respective first attachment zone being positioned at an upper corner of the front portion and said second attachment zone being positioned on the front portion closer to the line of symmetry of the absorbent article and along the propagating direction of the band portion when said absorbent article is worn by a wearer.

The inventive absorbent article provides a waist band based fastening system which is easily adjustable, by the wearer or any assisting personnel, around the waist of the wearer and provides a thorough fit of the absorbent article to wearer's, which are within a substantial interval as regards size and shape. The positioning of the first attachment zone at an upper corner of the front portion makes it possible to resist sagging and by utilizing the more rigid part of the absorbent article shear is primarily transferred within the body of the absorbent article, the body being the more rigid part of the absorbent article. Furthermore, the improved absorbent article enables an improved method of applying said absorbent article.

The method comprises the steps of joining respective first fastening means in an adjustable manner in a respective first attachment zone positioned on the substantially upper corner of the front portion of said absorbent article and when said first fastening means is joined in an appropriate position said second fastening means are being joined in a respective second attachment zone closer to the line of symmetry of the absorbent article for providing a holding effect in a primarily longitudinal direction propagating along the band portions.

The design of the improved absorbent article allows for an initial adjusting of the absorbent article by manipulating the first fastening means. Preferably, said step of joining said first fastening means in an adjustable manner comprises reconnection of said first fastening means.

Once the absorbent article is suitably positioned to the body of the wearer said second fastening means are being joined in a respective second attachment zone closer to the line of symmetry of the absorbent article for providing a holding effect in a primarily longitudinal direction propagating along the band portions.

Preferably, the band portions are applied tightly towards the second fastening means. Preferably, the step of joining said second fastening means comprises the step of attaching a tape portion which extends substantially longitudinal in relation to the propagation of the band portions.

When the absorbent article is applied for use to a wearer said first fastening means provides a sagging resisting effect in one primarily transverse direction in relation to the propagation of the band portions.

Having described the method of applying the absorbent article the benefits of the combination and positioning of the features of the absorbent article fastening system will be more apparent.

Advantageously, said first fastening means are reconnectable, thus allowing for initial adjustment and positioning of the absorbent article before final fixing of said absorbent article to the wearer is accomplished by said second fastening means.

More preferably, said first fastening means, when joined, provide stronger holding in a transverse direction in relation to the propagation of the band portions than in a direction along said band portions.

Said first fastening means is a mechanical fastener of hook-and-loop type, thus allowing for reconnection and yet providing a solid holding. Advantageously, at least said second fastening means comprises a tape.

Alternatively, said first fastening means comprises a reconnectable tape portion. According to another aspect of the invention said front portion comprises a landing zone which forms part of any one of said fastening means. Any of said fastening means comprises a reconnectable landing zone.

It is realized that the fastening system is especially well adapted for absorbent articles having band portions with a non-elastic characteristic due to its flexibility and ease in adjusting. An elastic material resumes to its original size and shape after stretch impact but is generally less cost efficient.

It is realized that the first and second fastener means may be designed in various ways within the claimed scope as long as they together provides sagging resistance and secure fit around the waist of the wearer.

### Brief Description of the Drawings

A currently preferred embodiment of the present invention will now be described in more detail, with reference to the accompanying drawings.

Fig. 1 is a plan view of a first aspect of an absorbent article according to an embodiment of the invention.

Fig. 2 is a plan view of a second aspect of an absorbent article according to an embodiment of the invention.

Fig. 3 is a plan view of a third aspect of an absorbent article according to an embodiment of the invention.

Fig. 4a is a perspective view of an absorbent article according to an embodiment of the invention, wherein the first attachment means are joined.

Fig. 4b is a perspective view of an absorbent article according to an embodiment of the invention, wherein the first second attachment means are joined.

### Detailed Description of Preferred Embodiments

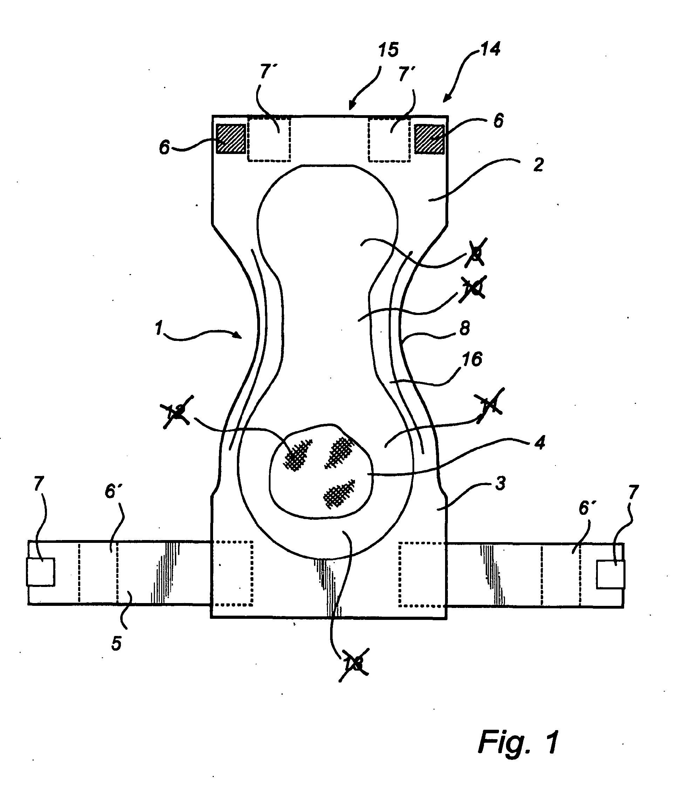

A first embodiment of the invention related to an absorbent article will be described in more detail in the following with reference to the accompanying drawings. Referring now to Fig. 1, wherein a first aspect of the fastening system is typically present in the form of a band portion 5, essentially of conventional wing type, of the absorbent article 1 to provide a fastening system for holding the absorbent article on the wearer.

The fastening arrangement is provided with a pair of band portions 5 propagating from a rear portion 3 and adapted to join said front portion 2 from respective side of the waist when said absorbent article 1 is worn by a wearer. Furthermore, said absorbent article 1 is provided with first fastening means 6 and second fastening means 7 for providing said joining. Said first fastening means 6, 6', when joined, provide a retaining effect against skewing of the front portion 2, and each said second fastening means 7, when joined, provides a pull resisting effect in a primarily band portion longitudinal direction propagating along the band portions 5.

Furthermore, said first fastening means 6 are joinable in a respective first attachment zone 14 on the front portion 2 of said absorbent article 1 and said second fastening means 7, 7' are joinable in a respective second attachment zone 15 closer to the line of symmetry of the absorbent article. The respective first and second attachment zone 14, 15 are arranged in a primarily longitudinal direction propagating along the band portions 5 in their intended use position.

Moreover, the first fastening means 6, 6' are reconnectable, thus allowing for initial adjustment of the absorbent article 1 before final positioning of said absorbent article 1 to the wearer is accomplished by said second fastening means 7. The first fastening means 6, when joined, provide more holding effect in one transverse direction in relation to the propagation of the band portions than in a direction along said band portions 5. The first fastening means 6, 6' comprises a hook portion which hooks extend substantially downwards in relation to a wearer when said absorbent article is positioned on the body of a wearer.

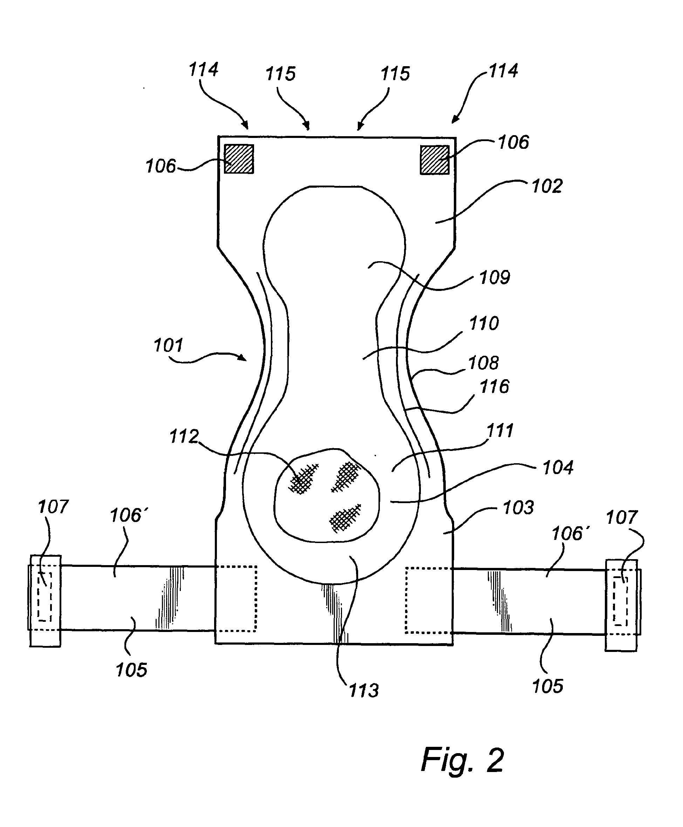

According to a second aspect of the invention which is disclosed in fig 2 the first fastening means 106, 106' is of hook-and-loop type. The first fastening means 106, 106' comprises a hook portion which hooks extend substantially downwards in relation to a wearer when said absorbent article is positioned on the body of a wearer.

The second fastening means comprises a reconnectable landing zone 107 being adapted to attach to the front portion second attachment zone 115 and on another side attach to said band portion 105 second fastening means. The reconnectable landing zone 107 is reconnectable on both sides and in its original position attached to the band portion 105 and covered by a plastic strip on the other side. When the plastic strip is removed the second fastening means is reconnectably connectable to said second attachment zone 115. The features that are similar to that of fig 1 are not repeated again.

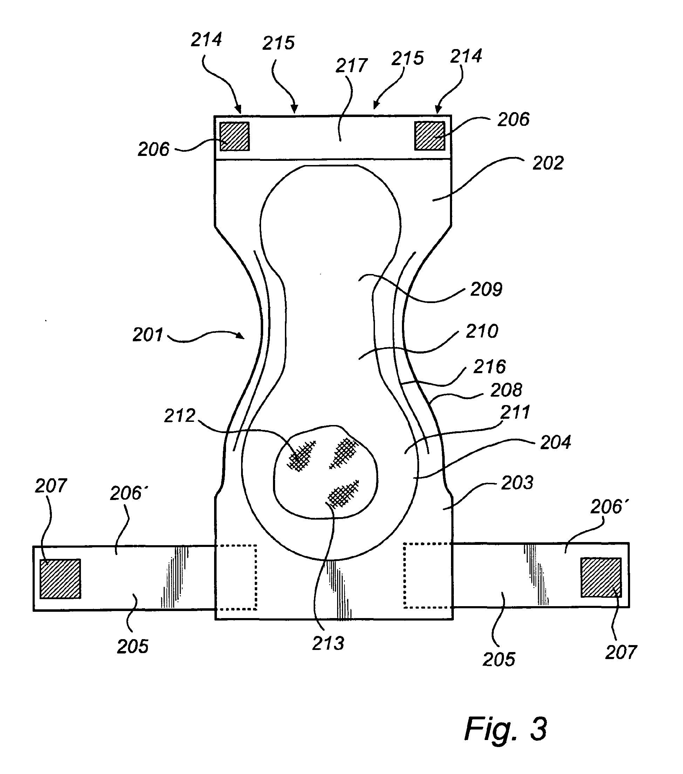

In fig 3 a third aspect of the invention is disclosed. The features that are similar to that of fig 1 are not repeated again. The first fastening means 206, 206' and second fastening means 207 is of hook-and-loop type. More advantageously, said second fastening means 207 comprises a hook portion which hooks are directed away from the symmetry line of the absorbent article 1 when said absorbent article is positioned on the body of a wearer.

In one alternative embodiment said front portion comprises a landing zone (not shown) which forms part of any one of said fastening means 6, 6', 7, 7'. The landing zone itself may also be reconnectable although not shown here.

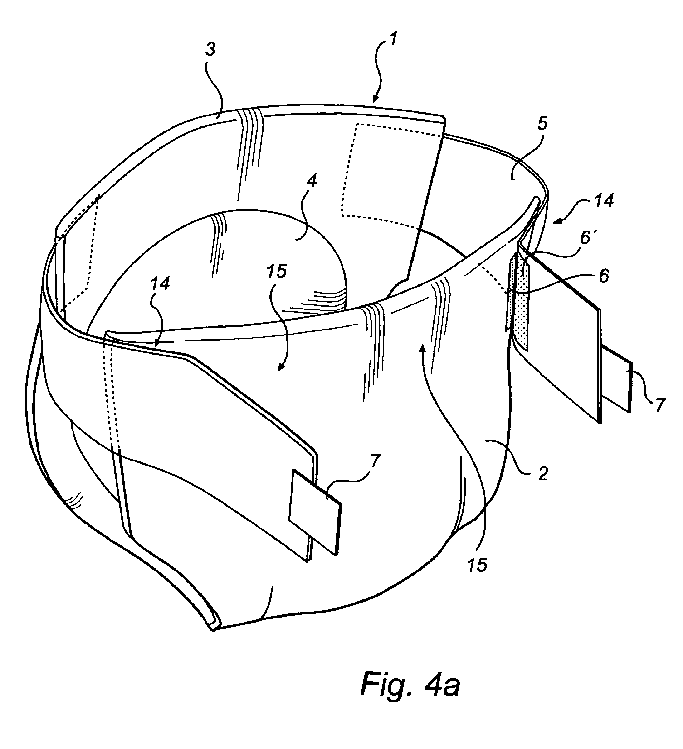

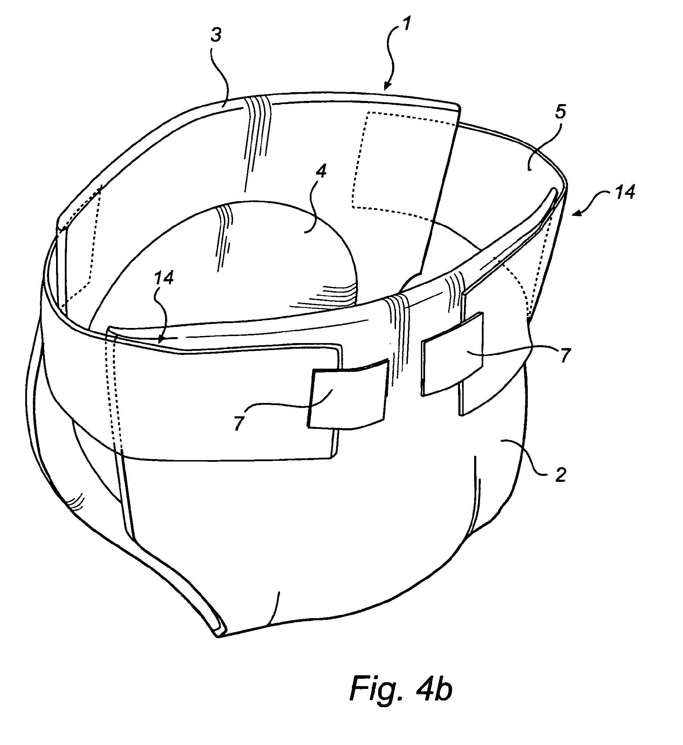

With reference to fig 4a and 4b an illustration of the method of applying said absorbent article is provided. When the absorbent article is applied to a wearer the fastener arrangement is joined by the first fastening means 6 at the corners of the front portion 2 which is denoted as a first attachment zone 14 of the absorbent article 1 and the second fastening means 7 are next to be joined by a second attachment zone 15. Before the second fasteners are joined the step of adjusting the absorbent article comfortably around the waist by means of the reconnectable first fastening means 6 preferably takes place. This is best illustrated by fig 4a.

In fig 4b the second fastening means 7 are joined to the front portion of the absorbent article and thus the absorbent article is secured around the waist.

It is realised from the above given examples that there exist various options for the skilled person to design a fastener arrangement that enables the desired functions by using a first and second fastening means and that this could be accomplished by combining the presented features in various ways.

## Claims

1. An absorbent article (1), such as an incontinence guard, comprising a front portion (2), a rear portion (3) and a crotch region (4) there between,
wherein the absorbent article has a longitudinal direction, extending from the front portion to the rear portion, a transversal direction, orthogonal to the longitudinal direction, and a line of symmetry extending along the longitudinal direction,
wherein the absorbent article is further provided with a pair of band portions (5) propagating from the rear portion (3) in the transversal direction of the absorbent article (1) and adapted to join said front portion (2) from respective side of the waist when said absorbent article (1) is worn by a wearer,
said each band portion (5) being reconnectably joinable to said front portion in a respective first attachment zone (14) of said absorbent article (1) by a first fastening means (6),
said each band portion (5) being joinable by a second fastening means (7) in a respective second attachment zone (15), said respective first attachment zone (14) being positioned at an upper corner of the front portion (2) and said second attachment zone (15) being positioned on the front portion (2) closer to the line of symmetry of the absorbent article (1) than the first attachment zone (15), and along the propagating direction of the band portion (5) when said absorbent article (1) is worn by a wearer, said first fastening means (6) being of hook-and-loop type,
**characterized in that**
the first fastening means (6) comprises a hook portion in which the hooks extend substantially downwards in relation to a wearer when said absorbent article is positioned on the body of a wearer.

2. The absorbent article (1) according to claim 1, wherein said first fastening means (6) are reconnectable, thus allowing for initial adjustment and positioning of the absorbent article (1) before final fixing of said absorbent article (1) to the wearer is accomplished by said second fastening means (7).

3. The absorbent article according to any one of claims 1-2, wherein said second fastening means (7) is of hook-and-loop type, and wherein the second fastening means comprises a hook portion in which the hooks are directed away from the line of symmetry of the absorbent article when the absorbent article is positioned on the body of a wearer.

4. The absorbent article (1) according to any one of claims 1-3, wherein at least said second fastening means (7) comprises a tape.

5. The absorbent article (1) according to any one of claims 1-4, wherein said front portion comprises a landing zone (217) which forms part of any one of said fastening means (6, 6', 7, 7').

6. The absorbent article (1) according to any one of claims 1-5, wherein any of said fastening means (6, 6', 7, 7') comprises a reconnectable landing zone (107).

7. The absorbent article (1) according to any one of claims 1-6, wherein said band portions (5) has a non-elastic characteristic.

## Patentansprüche

1. Absorbierender Artikel (1), wie ein Inkontinenzschutz, umfassend einen vorderen Abschnitt (2), einen hinteren Abschnitt (3) und dazwischen einen Schrittbereich (4),
wobei der absorbierende Artikel eine Längsrichtung, die sich vom vorderen Abschnitt zum hinteren Abschnitt erstreckt, eine Querrichtung, orthogonal zur Längsrichtung, und eine Symmetrielinie, die sich entlang der Längsrichtung erstreckt aufweist,
wobei der absorbierende Artikel ferner mit zwei Bandabschnitten (5) versehen ist, die vom hinteren Abschnitt (3) in Querrichtung des absorbierenden Artikels (1) verlaufen und dazu ausgebildet sind, sich mit dem vorderen Abschnitt (2) von einer entsprechenden Seite der Taille zu verbinden, wenn der absorbierende Artikel (1) von einem Träger getragen wird,
wobei jeder Bandabschnitt (5) wieder verschließbar mit dem vorderen Abschnitt in einer entsprechenden ersten Befestigungszone (14) des absorbierenden Artikels (1) durch ein erstes Befestigungsmittel (6) verbunden werden kann,
wobei der Bandabschnitt (5) durch ein zweites Befestigungsmittel (7) in einer entsprechenden zweiten Befestigungszone (15) verbunden werden kann, wobei die erste Befestigungszone (14) an einer oberen Ecke des vorderen Abschnitts (2) positioniert ist und die zweite Befestigungszone (15) am vorderen Abschnitt (2) näher zur Symmetrielinie des absorbierenden Artikels (1) als die erste Befestigungszone (15) und entlang der Erstreckungsrichtung des Bandabschnitts (5) positioniert ist, wenn der der absorbierende Artikel (1) von einem Träger getragen wird, wobei das erste Befestigungsmittel (6) von Haken-und-Ösen-Typ ist,
**dadurch gekennzeichnet, dass**
das erste Befestigungsmittel (6) einen Hakenabschnitt umfasst, in dem sich die Haken im Wesentlichen abwärts in Bezug auf einen Träger erstrecken, wenn der Artikel am Körper eines Trägers positioniert ist.

2. Absorbierender Artikel (1) nach Anspruch 1, wobei die ersten Befestigungselemente (6) wieder verschließbar sind, wodurch eine anfängliche Anpassung und Positionierung des absorbierenden Artikels (1) möglich ist, bevor der absorbierende Artikel (1) endgültig durch das zweite Befestigungselement (7) am Träger fixiert wird.

3. Absorbierender Artikel nach einem der Ansprüche 1 bis 2, wobei das zweite Befestigungselement (7) vom Haken-und-Ösen-Typ ist und wobei das zweite Befestigungselement einen Hakenabschnitt umfasst, in dem die Haken von der Symmetrielinie des absorbierenden Artikels weg gerichtet sind, wenn der absorbierende Artikel am Körper eines Trägers positioniert ist.

4. Absorbierender Artikel (1) nach einem der Ansprüche 1 bis 3, wobei zumindest das zweite Befestigungselement (7) ein Band umfasst.

5. Absorbierender Artikel (1) nach einem der Ansprüche 1 bis 4, wobei der vordere Abschnitt eine Anlegezone (217) umfasst, die Teil eines der Befestigungselemente (6, 6', 7,7') bildet.

6. Absorbierender Artikel (1) nach einem der Ansprüche 1 bis 5, wobei jedes der Befestigungselemente (6, 6', 7,7') eine wieder verschließbare Anlegezone (107) umfasst.

7. Absorbierender Artikel (1) nach einem der Ansprüche 1 bis 6, wobei die Bandabschnitte (5) eine nicht elastische Eigenschaft haben.

## Revendications

1. Article absorbant (1), de type serviette pour incontinents, comprenant une partie avant (2), une partie arrière (3) et une partie entrejambe (4) entre elles,
cet article absorbant ayant un sens longitudinal s'étendant de la partie avant à la partie arrière, un sens transversal orthogonal par rapport au sens longitudinal et une ligne de symétrie s'étendant dans le sens longitudinal,
cet article absorbant étant en outre pourvu d'une paire de parties en bande (5) se diffusant depuis la partie arrière (3) dans le sens longitudinal de l'article absorbant (1) et aptes à rejoindre ladite partie avant (2) depuis le côté respectif de la taille lorsque ledit article absorbant (1) est porté par un utilisateur,
chaque dite partie en bande (5) pouvant être assemblée de manière reconnectable à ladite partie avant dans une première zone de fixation respective (14) dudit article absorbant (1) par un premier moyen de fixation (6),
chaque dite partie en bande (5) pouvant être assemblée par un second moyen de fixation (7) dans une seconde zone de fixation respective (15), ladite première zone de fixation (14) étant positionnée à un coin supérieur de la partie avant (2) et ladite seconde zone de fixation (15) étant positionnée sur la partie avant (2) plus près de la ligne de symétrie de l'article absorbant (1) que la première zone de fixation (15) et le long de la zone de diffusion de la partie en bande (5) lorsque ledit article absorbant (1) est porté par un utilisateur, ledit premier moyen de fixation (6) étant de type Velcro,
**caractérisé en ce que**
le premier moyen de fixation (6) comprend une partie à crochets dans laquelle les crochets s'étendent sensiblement vers le bas par rapport à un utilisateur lorsque ledit article absorbant est positionné sur le corps d'un utilisateur.

2. Article absorbant (1) selon la revendication 1, dans lequel le premier moyen de fixation (6) est reconnectable, ce qui permet l'ajustement et le positionnement initial de l'article absorbant (1) avant que la fixation finale dudit article absorbant (1) sur l'utilisateur soit faite par ledit moyen de fixation (7).

3. Article absorbant selon l'une quelconque des revendications 1 et 2, dans lequel le second moyen de fixation (7) est de type Velcro et le second moyen de fixation comprend une partie à crochets dans laquelle les crochets sont détournés de la ligne de symétrie de l'article absorbant lorsque l'article absorbant est positionné sur le corps d'un utilisateur.

4. Article absorbant (1) selon l'une quelconque des revendications 1 à 3, dans lequel au moins le second moyen de fixation (7) comprend un ruban.

5. Article absorbant (1) selon l'une quelconque des revendications 1 à 4, dans lequel ladite partie avant comprend une zone de dépôt (217) qui fait partie intégrante de l'un quelconque desdits moyens de fixation (6, 6', 7, 7').

6. Article absorbant (1) selon l'une quelconque des revendications 1 à 5, dans lequel l'un quelconque desdits moyens de fixation (6, 6', 7, 7') comprend une zone de dépôt reconnectable (107).

7. Article absorbant (1) selon l'une quelconque des revendications 1 à 6, dans lequel lesdites parties en bande (5) ont une caractéristique non élastique.
